# EUROPEAN PATENT SPECIFICATION

(11) **EP 2 073 735 B1**
(45) Date of publication and mention of the grant of the patent: **24.11.2010**
(21) Application number: 07805563.9
(22) Date of filing: 06.09.2007
(51) Int. Cl.: A61B 18/02

(54) **DEVICE FOR COMBINED TREATMENT**
VORRICHTUNG FÜR KOMBINIERTE BEHANDLUNG
DISPOSITIF POUR POLYTHÉRAPIE

(30) Priority: 08.09.2006 US 824967 P
(43) Date of publication of application: 01.07.2009
(73) Proprietor: Arbel Medical Ltd., 20692 Yokenam (IL)
(72) Inventor: TOUBIA, Didier, 43559 Ra'anana (IL); LEVIN, Alexander, 30500 Binyamina (IL); KAGANOVICH, Miron, 31999 Haifa (IL); EYAL-WALDMAN, Lilach, 76850 Moshav Meishar (IL)
(74) Representative: Messulam, Alec Moses
(86) International application number: PCT/IL2007/001103
(87) International publication number: WO 2008/029408

(56) References cited:
- WO-A-01/37919
- WO-A-01/97702
- WO-A-96/39960
- WO-A-2006/127467
- WO-A-2007/086056
- US-A1- 2003 181 897

## Description

### FIELD OF THE INVENTION

The present invention is directed to providing a device for improved treatment based on the combination of physical therapy, preferably a cryosurgical system, and the delivery of an active agent to the tissue being treated for complementing or enhancing the action of the physical therapy.

### BACKGROUND OF THE INVENTION

Cryosurgery is defined as a "surgery in which diseased or abnormal tissue (as a tumor or wart) is destroyed or removed by freezing. " (Source: Webster medical dictionary). Cryosurgery uses a rigid "cryoprobe", or a flexible "cryocatheter," to destroy internal or superficialtissues.

Cryosurgery is a minimally invasive technique. The product is generally composed of a console (including a cryogen container or balloon with highly pressurized gas) and a control system, and a probe called a cryoprobe. This probe is in fluid communication with the console. Alternatively, the cryoprobe may be replaced by a flexible catheter called "ciyocatheter".

During the procedure, the cryoprobe is positioned in the body tissue through a tiny incision, which is typically selected with the assistance of a suitable imaging technique. An adapted freezing-thawing cycle at the cryoprobe's tip ("cryotip") ablates internal tissue. The dead cells are eliminated from the body over time through a natural and spontaneous process called lysis. As the tissue is not cut, there are fewer risks of bleeding, infection, and side effects. Recovery is generally short.

Therapeutic drug-device combination products are an area of intense interest and unlimited potential from a clinical as well as an investment point of view. Devices, in general, are used to treat conditions rather than cure them. Drugs and biologics also generally treat disease, although some products provide cures. The combining of two treatment approaches, drugs/biologics and devices, should enhance the quality of treatment by increasing efficacy and reducing side effects. In some cases, the combination may, in fact, effect a cure.

The total market for drug-device combinations worldwide was valued at $5.4 billion in 2004 and is expected to rise at an average annual growth rate (AAGR) of 13.6% to $11.5 billion in 2010.

The background art includes several attempts to combine cryosurgical, or other physical therapies, with a biological agent.

For example Ikekawa S, Ishihara K, Tanaka S, Ikeda S studied the combined effect of cryosurgery and anticancer drugs (cryochemotherapy) in an experimental B16 melanoma/BDF1 tumor system. They showed that the vascular volume and vascular permeability of both the normal vessels and the tumor vessels greatly increased immediately after cryosurgery, and their vascular volume decreased to less than the normal level within a few hours. The anticancer drugs, peplomycin and adriamycin, were administered intraperitoneally in combination with cryosurgery.

Other researchers from the Institute Gustave-Roussy in France and Boris Rubinsky from the University of California, Berkeley found that freezing cancer cells in test tubes made them far more vulnerable to attack by bleomycin, a potent anti-cancer drug also known by the brand name Blenoxane. Cryosurgery - freezing cells to destroy them - and bleomycin are approved treatments currently used separately for cancer patients. But researchers of the study say that combining the two therapies may eventually lead to a powerful new form of cancer treatment that targets malignant cells while leaving healthy tissue unharmed.

US Patent Application No. US 11/087,156 discloses therapeutic methods for treating tumors and cancerous tissues by first - inducing necrosis or apoptosis (e.g. cryotherapy, chemotherapy, radiation therapy, or others), and then delivering one or more antigen presenting cells (e.g. autologous dendritic cells) intratumorally or proximate to the tumor or cancerous tissue after a selected period of time sufficient for the bio-availability of the liberated cancer-specific antigens resulting from necrosis or apoptosis to be near or at maximum value.

Nordouist (US Publication 2005/0106153) discloses an invention combining physical and immunologic therapies for the treatment of neoplasms by conditioning a targeted neoplasm with an immunoadjuvant (also called immunomodulator or immunopotentiator) and then physically destroying the conditioned neoplasm. A number of physical therapies can be used to achieve the physical destruction of the conditioned tumor mass, including cryotherapy.

Other biological agents are used to enhance to lethal effect of cryogenic cooling. For example, Rubinsky (US patent No. 5,654,279) proposes to enhance cell and tissue destruction following cryosurgery by perfusion of the cells with thermal hysteresis proteins prior to the cryogenic freezing. The effect of the proteins is to promote the growth of ice crystals in the intra-cellular fluid which destroy the cell by piercing the cell membrane.
Further methods and devices are disclosed in WO 01/37919A and WO96/39960 A.

### SUMMARY OF THE INVENTION

The background art does not teach or suggest a cryoprobe combined with a syringe for more effective delivery of biological agents or drugs at the site of the cryosurgical treatment. The background art also does not teach or suggest such a cryoprobe for providing more exact injection of the one or more active agents with respect to the location of tissue freezing and/or for providing a shorter time required for the injection.

The present invention according to claim 1 overcomes these drawbacks of the background by providing, in some embodiments, a cryoprobe for supporting administration of an active agent to a location of cryogenic treatment. The cryoprobe may optionally be combined with a syringe to form a cryoprobe system as described herein; alternatively and optionally, the syringe may be external to the cryoprobe.

Said cryoprobe is adapted to be employed in a method for treatment comprising administering an active agent to a precise site of cryotherapy,
including the local delivery of at least one proteolytic enzyme or other enzymes, which increases the rate of shrinking of the treated lesion (i.e. tumor) following the physical treatment.

At least one or more chemotherapeutic agents enhancing the effect of the cryotherapy may be locally delivered immediately before or after the physical treatment.

Said cryoprobe is suitable for the local delivery of at least one vasoconstrictive agent immediately before a cryotherapy or a cryosurgical therapy. The narrowing of blood vessels limits the heating of the tissue during freezing, and makes the therapy more effective and/or rapid.

A solution, preferably an aqueous solution, injected into the tissue prior to the cryosurgical treatment can serve for enhancement of the cryo-damaging effect intended to destroy a significant fraction of this tissue.

Furthermore, said cryoprobe may be employed in a method for delivery of a combination of at least two of the above-mentioned categories of biological agents, drugs or liquids.

Optionally, the active agent may be injected before, during or after cryotreatment.

It should be noted that the injected material can contain radioactive elements; in such a way, the proposed method may present a combination of cryosurgery with brachytherapy, or cryosurgery, brachytherapy and pharmacological treatment. The methods described herein do not form part of the invention.

### BRIEF DESCRIPTION OF THE DRAWINGS

FIG. 1a shows an axial cross-section of a cryoprobe with a set of stationary adjacent needles.

FIG. 1b, FIG. 1c and FIG. 1d show three transversal cross-sections of the combination: cryoprobe-syringe in three different places, corresponding to lines A-A, B-B and C-C in FIG. 1a.

FIG. 2 shows an axial cross-section of a cryoprobe with a set of adjacent needles and a mechanism for the needles' displacement.

FIG. 3 shows an axial cross-section of a cryoprobe with a set of adjacent needles and a different mechanism for the needles' displacement.

FIG. 4 shows an axial cross-section of a cryoprobe with a set of adjacent syringe and a proximal mechanism for successive displacement of the needles of the syringe with following drug injection and backward displacement of the syringes' needles.

### DESCRIPTION OF THE PREFERRED EMBODIMENTS

In some cases, the method features local delivery (at the site of a cryosurgical treatment) of at least one enzyme, such as a proteolytic enzyme, following a cryosurgical treatment. Such enzyme speeds up the shrinking of the treated lesion or tissue (i.e. tumor).

In another case the method features the combination of physical therapy including, but not limited to, cryosurgery with the delivery of a chemotherapeutical or cytotoxic agent in order to enhance the lethal effect of freezing on cells and tissue.

In another variant, an aqueous solution injected into the tissue prior to the cryosurgical treatment can serve for enhancement of the cryo-damaging effect intended to destroy a significant fraction of this tissue.

In another variant the injected liquid may optionally contain one or more radioactive elements; in such a way that the proposed method may present a combination of cryosurgery with brachytherapy, or cryosurgery, brachytherapy and pharmacological treatment.

All of the above active ingredients, including a solution such as an aqueous solution for example, are termed herein "active agents" .

In another variant, the method includes the delivery of a combination of two or more of the above-mentioned categories of biological agents or drugs.

EXAMPLE 1

ILLUSTRATIVE METHOD OF TREATMENT

This Example relates to illustrative methods of treatment . An active agent is preferably administered to the tissue receiving cryotherapy before, during or after such cryotherapy is performed (or a combination thereof). The active agent may optionally be administered in the form of a composition as described in greater detail below. The active agent is preferably administered at the substantially same location as the site receiving cryotherapy, such that the distance between the center of the area receiving cryotherapy and the center of the area receiving the active agent is optionally and preferably a minimal distance. The active agent is preferably administered by injection.

The method may optionally comprise a combination in which the active agent is administered more than once during treatment (for example, before and during cryotherapy, before and after cryotherapy, during and after cryotherapy and so forth). Different active agents may also optionally be administered at different times of the cryotherapeutic process. A different active agent may optionally be administered before cryotherapy than an active agent administered after cryotherapy, for example.

The order may optionally be determined according to one or more characteristics of the active agent itself. For example, if the active agent is sensitive to cold temperature, then it may be preferable to avoid administration during cryotherapy and/or to otherwise adjust administration of the active agent to overcome this sensitivity.

EXAMPLE 2

ILLUSTRATIVE ACTIVE AGENTS AND COMPOSITIONS

Illustrative substances (compositions) for use with the device of the present invention include but are not limited to any active agent as described herein, including enzymes, chemotherapeutic agents, various types of drugs, biologic agents (including but not limited to proteins, polynucleotides, siRNAs, antibodies, peptides and so forth), radioactive substances, and solutions which are otherwise insert.

With regard to enzymes, the enzyme or enzymes to be delivered may include one or more of the following, but are not limited to: (a) proteolytic enzymes such as_Hyaluronidase, Pancreatin, Pepsin, Papain, Dispase, Trypsin, Subtilisin for example; (b) enzymes used for tissue debridment such as Collagenase, Papain / urea combination, Fibrinolysin in combination with deoxyribonuclease (DNase) or not, Streptokinase / streptodornase, Krill enzyme - new multi-enzyme preparation isolated from Antarctic shrimp-like organisms, for instance; (c) thrombolytic (fibrinolytic) agents such as_Alteplase, Anistreplase, Streptokinase, Urokinase (Abbokinase®) for instance.

By way of example, collagenase is an enzyme that has the specific ability to digest collagen. Still by the way of example, the proteolytic enzyme fibrinolysin targets fibrin. Fibrin degradation products stimulate macrophages to release growth factors into the wound bed.

Chemotherapy drugs used in some embodiments of the present invnetion may include, but are not limited to: (a) Alkylating agents such as Cyclophosphamide, Chlorambucil, Melphalan for instance; (b) Antimetabolites such as Methotrexate, Cytarabine, Fludarabine, 6-Mercaptopurine, 5-Fluorouracil; (c) Antimitotics such as Vincristine, Paclitaxel, Vinorelbine; (d) Topoisomerase inhibitors such as Doxorubicin, Irinotecan for instance; (e) Platinum derivatives such as Cisplatin, Carboplatin (f) Hormonal therapies such as Tamoxifen, Bicalutamide; (g) Monoclonal antibodies such as Rituximab, Trastuzumab, Gemtuzumab ozogamicin; (h) Biologic response modifiers such as Interferon-alpha; (i) Differentiating agents such as Tretinoin. Optionally, in addition or alternatively, steroid drugs may be used as in high doses they are potent chemotherapy drugs. Non-limiting examples of steroid drugs include corticosteroids, androgens, estrogens, and progestagens (sex steroids) and anabolic steroids.

By way of example, a hormonal drug such as Tamoxifen blocks estrogen action in breast cancer and monoclonal antibody such as Trastuzumab blocks the growth factor receptor on breast cancer cells.

In yet another variant, a vasoconstrictive agent is delivered locally in the site to be treated by cryosurgery before the cryosurgical treatment is carried out. A vasoconstrictive agent is any agent that causes a narrowing of blood vessels: nicotine or epinephrine or norepinephrine or angiotensin or vasopressin or adrenalin or prostaglandin F_{2α} or felypressin, or S-ethylisothiourea (S-EITU) or Somatostatin and its analogues, such as octreotide or a combination of two or more of the mentioned agents or drugs, for example. The induced local vasoconstrictive action limits the blood circulation and then the heating of the site to be treated during the treatment. As a way of consequence, the freezing of the site is faster and the temperature reached is lower than when the cryosurgical device is used alone.

A "solution which is otherwise inert" relates to any solution which does not contain an additional therapeutic substance beyond the solution itself. Non-limiting examples of such a solution include a saline solution and an ethanol solution.

The method proposed herein encompasses any active agent disclosed in Desai (US Publication No. 2005/025503).

The composition to be administered is preferably in a form selected from the group consisting of a liquid, a gel, a semi-solid or a gas, or a combination thereof.

Hereinafter, the phrases "physiologically acceptable carrier" and "phasmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, vegetable oils and polyethylene glycols.

Techniques for formulation and administration of drugs may be found in "Remington's Pharmaceutical Sciences," Mack Publishing Co., Easton, PA, latest edition.

Pharmaceutical compositions may be manufactured by processes well known in the art, e.g., by means of conventional mixing, dissolving, emulsifying, entrapping or lyophilizing processes.

Pharmaceutical compositions for use in accordance with the present invention may be formulated in conventional manner using one or more physiologically acceptable carriers comprising excipients and auxiliaries, which facilitate processing of the active ingredients into preparations which, can be used pharmaceutically.

For injection, the active ingredients may be formulated in aqueous solutions, preferably in physiologically compatible buffers such as Hank's solution, Ringer's solution, or physiological salt buffer.

Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

Pharmaceutical compositions suitable for use in context of the proposed method include compositions wherein the active ingredients are contained in an amount effective to achieve the intended purpose. More specifically, a therapeutically effective amount means an amount of active ingredients effective to prevent, alleviate or ameliorate symptoms of disease or prolong the survival of the subject being treated.

Determination of a therapeutically effective amount is well within the capability of those skilled in the art.

Table 1 below lists some exemplary, illustrative active agents which may optionally be used for treatment of breast cancer, in terms of adjuvant and neoadjuvant therapy. It should be noted that the dosing amounts and regimen given is for systemic administration; it may therefore optionally be preferred to adjust the dosing amount and/or regimen for local and/or site specific administration, as could easily be done by one of ordinary skill in the art.

Chemotherapy can be used as adjuvant therapy after breast conservation therapy or mastectomy. As such, chemotherapy reduces the risk of breast cancer recurrence. Chemotherapy can also be used as the main treatment for women whose cancer has already spread outside the breast and underarm area at the time it is diagnosed, or if it spreads after initial treatments. The length of these treatments is not definite, but depends on whether the cancer shrinks and how much it shrinks. Chemotherapy given before surgery is called neoadjuvant therapy. The major benefit of neoadjuvant chemotherapy is that it can shrink large cancers so that they are small enough to be removed by lumpectomy instead of mastectomy. Another possible advantage of neoadjuvant chemotherapy is that doctors can see how the cancer responds to chemotherapy.

The chemotherapy is given in cycles, with each period of treatment followed by a recovery period. The usual course of chemotherapy lasts between 3 to 6 months. In most cases, chemotherapy is most effective, either as an adjuvant or neoadjuvant therapy, when combinations of more than one chemotherapy drug are used together. The chemotherapy begins on the first day of each cycle, and then the body is given time to recover from the effects of chemotherapy. The chemotherapy drugs are then repeated to start the next "cycle." The time between giving the chemotherapy drugs is generally 2 or 3 weeks and varies according the specific chemotherapy drug or combination of drugs.

EXAMPLE 3

ILLUSTRATIVE DEVICE FOR DELIVERING AN AGENT TO THE SITE OF CRYOSURGICAL TREATMENT

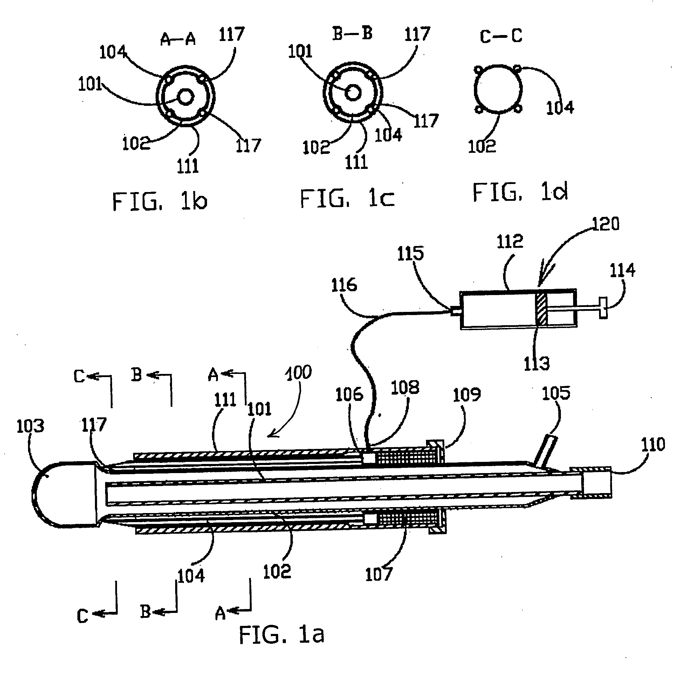

FIG. 1a, FIG. 1b, FIG. 1c and FIG. 1d show axial and transversal cross-sections of an illustrative cryoprobe 100 with a set of stationary adjacent needles.

Cryoprobe 100 preferably features a central supply lumen 101 with an inlet connection 110; a (preferably cylindrical) longitudinal shaft 102, which is provided with longitudinal grooves 117 diminishing to a zero or near zero dimension at their distal sections; and a cryotip 103. Central supply lumen 101 and longitudinal shaft 102 are preferably located within an external shaft 111.

A chamber 106, which is optionally angular, is preferably located within external shaft 111, adjacent to longitudinal shaft 102. Chamber 106 is for receiving an agent or agents to be administered to the site of cryotherapy. Chamber 106 features an inlet connection 108 and a (preferably annular) insert 107 for determining the position of chamber 106.

A plurality of needles 104 are positioned in the longitudinal grooves 117 of the longitudinal shaft 102 and are also preferably connected to, attached to or otherwise joined with chamber 106. More preferably needles 104 are syringe needles and are in fluid communication with chamber 106.

Inlet connection 108 is preferably connected to a barrel 112 of a syringe 120 situated external to cryoprobe 100. As described in greater detail below, syringe 120 preferably also features a piston 113 with a handle 114 for receiving pressure from a person administering the contents of syringe 120 (not shown). Barrel 112 is preferably connected to a flexible lumen 116 through a syringe outlet 115; flexible lumen 116 is in turn preferably connected to inlet connection 108 of chamber 106, such that chamber 106 is in fluid communication with barrel 112.

A proximal lid 109 closes the longitudinal shaft 102. Proximal lid 109 also pushes onto insert 107 when force is applied, which then pushes onto ring which then pushes on chamber 106. Chamber 106 becomes displaced, as does needle 104. Any substance or material within chamber 106 may optionally leave chamber 106 passively into needle 104 and/or due to pressure from having more material enter from barrel 112, as for example if piston 113 is depressed.

An outlet connection 105 which is connected to longitudinal shaft 102 permits release of the evaporated cryogen into the atmosphere from the internal space of the longitudinal shaft 102.

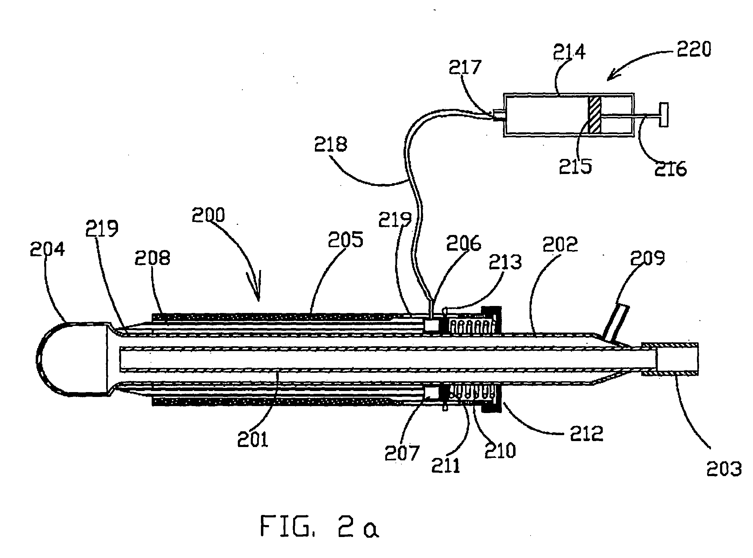

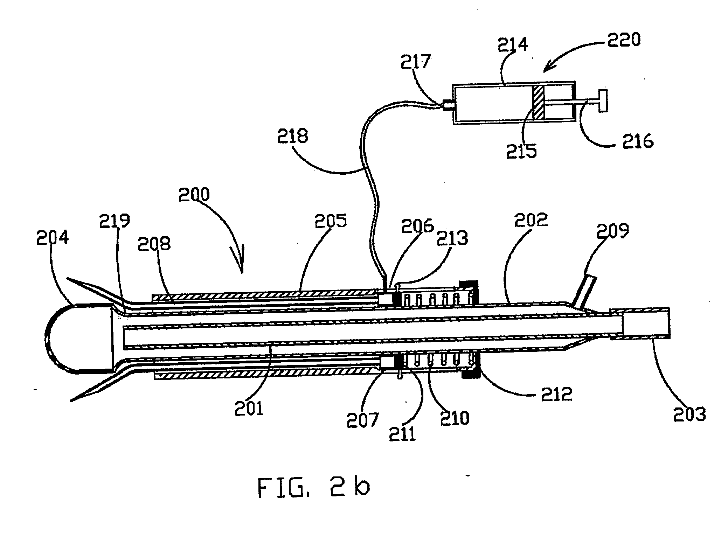

FIG. 2a and FIG. 2b show an axial cross-section of a cryoprobe 200 with a plurality of needles 208, a chamber 207 which is optionally annular, and a mechanism for displacement of needles 208 and chamber 207. The cryoprobe 200 comprises: a central supply lumen 201 with an inlet connection 203 for supply of a liquid cryogen; a longitudinal shaft 202, which is provided with longitudinal grooves 219 diminishing to a zero or near zero dimension at their distal sections; and cryotip 204. Central supply lumen 201 and longitudinal shaft 202 are preferably located within an external shaft 205. An outlet connection 209 at the proximal section of cylindrical longitudinal shaft 202 serves for release of the evaporated cryogen into the atmosphere. The distal end of the cylindrical longitudinal shaft 202 is sealed by cryotip 204.

Chamber 207 again preferably features an inlet connection 206 and needles 208. Inlet connection 206 is preferably connected to a barrel 214 of a syringe 220 situated external to cryoprobe 200. As described in greater detail below, syringe 220 preferably also features a piston 215 with a handle 216 for receiving pressure from a person administering the contents of syringe 220 (not shown). Barrel 214 is preferably connected to a flexible lumen 218 through a syringe outlet 217; flexible lumen 218 is in turn preferably connected to inlet connection 206. Needles 208 are positioned in the aforementioned longitudinal grooves 219 of the cylindrical longitudinal shaft 202. Needles 208 are preferably in fluid communication with chamber 207 and are also preferably syringe needles.

Adjacent to chamber 207, a washer 211 with handle 213 serves for displacement of chamber 207 and hence also of needles 208. Additional pressure is placed on washer 211 through a spring 210, which is preferably an extension spring for preventing or resisting displacement of chamber 207. External shaft 205 is preferably provided with a slot 219 for positioning and shifting the above-mentioned inlet connection 206 and handle 213.

As described above, inlet connection 206 of the chamber 207 is in fluid communication through lumen 218 with syringe outlet 217 of barrel 214 of the syringe. Upon application of pressure to handle 216 of syringe 220, piston 215 is moved into barrel 214, causing the contents of barrel 214 to move into lumen 218 and hence into chamber 207. Handle 213 of cryoprobe 220 may then be depressed, causing the contents of chamber 207 to become displaced into needles 208. Needles 208 also become displaced downward and outward over cryotip 204 (see Figure 2B). Before, during and/or after such displacement, cryogen enters through inlet connection 203 to central supply lumen 201, thereby permitting an iceball to form at needles 208. The contents of chamber 207 are therefore allowed to enter the area to be treated. Should it be desirable to administer the contents after placement of the needles 208, then the above displacement of handle 213 may optionally be performed first, followed by displacement of handle 216 to administer the contents of syringe 220.

A proximal lid 212 closes the external shaft 205 at its proximal end.

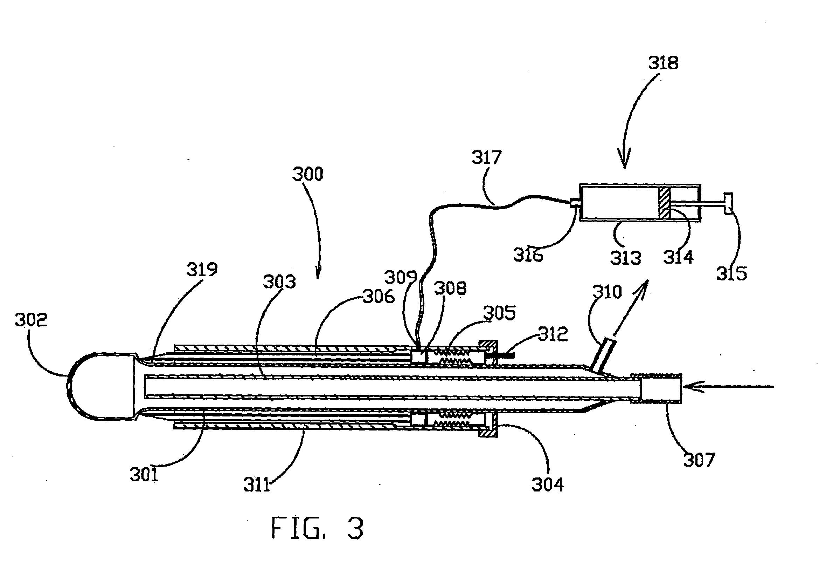

FIG. 3 shows an axial cross-section of a cryoprobe with a plurality of needles, an annular chamber and a pneumatic mechanism for the needles' displacement. The cryoprobe 300 comprises: a central supply lumen 303 with an inlet connection 307 for supply of a liquid cryogen; a longitudinal shaft 301, which is provided with longitudinal grooves 319 diminishing to a zero or near zero dimension at their distal sections; and cryotip 302. Central supply lumen 303 and longitudinal shaft 301 are preferably located within an external shaft 311. An outlet connection 310 at the proximal section of longitudinal shaft 301 serves for release of the evaporated cryogen into the atmosphere. The distal end of the longitudinal shaft 301 is sealed by cryotip 302.

Chamber 308 preferably features an inlet connection 309 and needles 306, which are preferably connected to a barrel 313 of a syringe 318 situated external to cryoprobe 300. As described in greater detail below, syringe 318 preferably also features a piston 314 with a handle 315 for receiving pressure from a person administering the contents of syringe 318 (not shown). Barrel 313 is preferably connected to a flexible lumen 317 through a syringe outlet 316; flexible lumen 317 is in turn preferably connected to inlet connection 309. Needles 306 are positioned in the aforementioned longitudinal grooves 319 of the longitudinal shaft 301.

In this embodiment, a pneumatic double-bellows cylinder 305 with a proximal inlet-outlet connection 312 through which air enters, preferably under force or pressure, is preferably joined with the proximal face plane of the chamber 308. This arrangement enables displacement of the chamber 308 with needles 306, preferably both forward and backward, by change of pneumatic pressure in the pneumatic double-bellows cylinder 305 through any mechanism which is know in the art, for example through an external controller of some type.

A proximal lid 304 closes the external shaft 31 at its proximal end, which is provided with an opening for positioning the aforementioned inlet-outlet connection 312.

As described above with regard to Figures 2a and 2b, the operation of cryoprobe 300 is similar, except that the needles 306 and the contents of chamber 308 are displaced through changing the pressure in the pneumatic double-bellows cylinder 305, rather than through manipulation of handle 213 as for the embodiment shown in Figure 2.

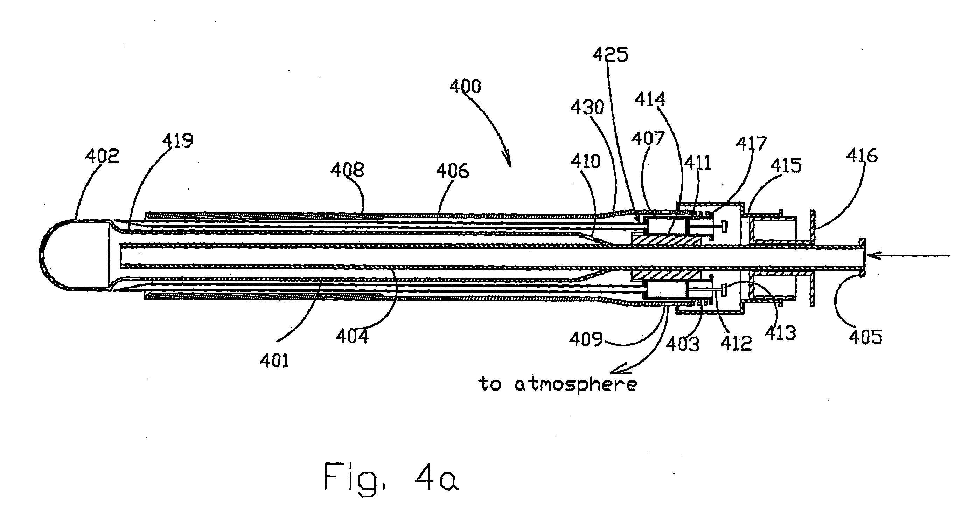

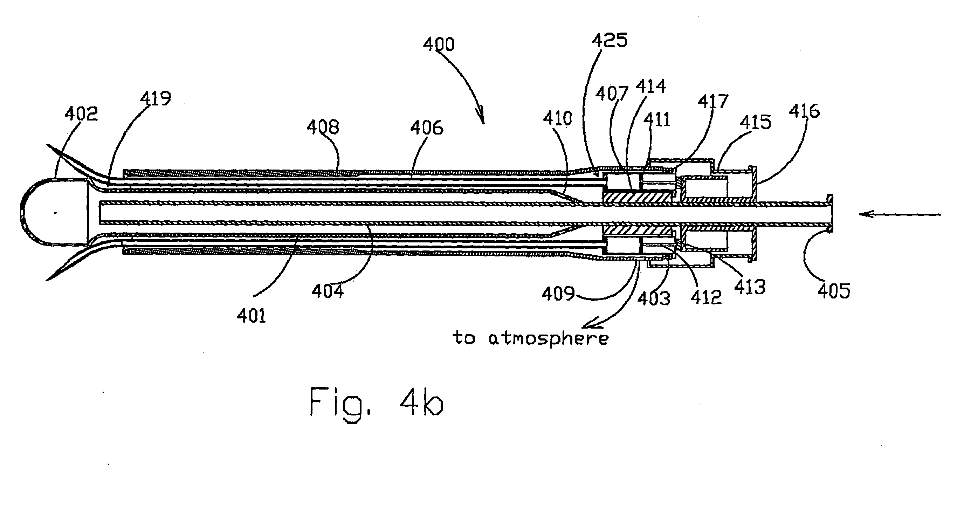

FIG. 4 shows an axial cross-section of a cryoprobe system, which also features an adjacent (or preferably set of syringes) and a proximal mechanical means for successive displacement of the needles of this syringe forward, followed by injection of the syringe contents and backward displacement of the syringe(s).

The cryoprobe system 400 features a central supplying lumen 404 with an inlet connection 405 for receiving cryogen. A longitudinal shaft 401, which is preferably cylindrical, preferably at least partially surrounds central supplying lumen 404 and is provided with a plurality of longitudinal grooves 419 diminishing to a zero or near zero dimension at their distal sections and also a plurality of openings 410 at its proximal section for removal of cryogen exhaust gases. The proximal end of the longitudinal shaft 401 is sealed with the proximal section of the central supplying lumen 404. The tip of the central supplying lumen 404 is turn sealed with a cryotip 402.

Cryoprobe system 400 also preferably features an external shaft 408 for surrounding lumen 404 and longitudinal shaft 401. Within external shaft 408, preferably a set of syringes 425 is situated such that a needle 406 of each syringe 425 is situated in the aforementioned longitudinal grooves 419 of longitudinal shaft 401. In addition, each syringe 425 preferably comprises a barrel 407, which is positioned in the recesses of bushing 414. Bushing 414 is arranged, in turn, on the proximal section of the central supplying lumen 404.

Each syringe 425 also features a piston 411 and flanging 417 for pushing against bushing 414. Each syringe 425 also features a handle 412 and button 413. External shaft 408 preferably covers the majority of needles 406 (except for their distal sections upon displacement).

An intermediate actuating member 415 closes the external sheath 408 at its proximal sections with possibility of its displacement along this external sheath 408. A proximal lid 416 closes the intermediate actuating member 415 at its proximal end; this proximal lid 416 is provided with the central opening for passage of central supplying lumen 404. Forward displacement of this proximal lid 416 initially displaces needles 406 and then preferably subsequently causes injection of a biologically active substance contained in barrels 407 into the treated tissue. Backward displacement of the needles 406 is provided by a helical spring 403, which preferably resists forward displacement of the needles 406.

While the invention has been described with respect to a limited number of embodiments, it will be appreciated that many variations, modifications and other applications of the invention may be made

Persons skilled in the art will appreciate that the present invention is not limited to what has been particularly shown and described hereinabove. Rather the scope of the present invention is defined by the appended claims and includes both combinations and sub combinations of the various features described hereinabove

In the claims, the word "comprise", and variations thereof such as "comprises", "comprising" and the like indicate that the components listed are included, but not generally to the exclusion of other components.

**Table 1**

| **Group** | **Subgroup** | **Target site** | **Drug name -generic** | **Brand name** | **Activity** | **Administration** | **Dosage** |
|---|---|---|---|---|---|---|---|
| Immuno-therapy | | HER2 receptor (of the HER2/neu gene) | Trastuzumab | Herceptin | Monoclonal antibody that blocks the HER2 receptor in cancer cells | IV | Initial dose 4 mg/kg, then 2 mg/kg once a week. |
| | | | Lapatinib | Tykerb | Kinase inhibitor | PO | 1250 mg daily |
| | | vascular endothelial growth factor (VEGF) | Bevacizumab | Avastin | Monoclonal antibody against angiogenesis | IV | 5 mg/kg every 14 days. |
| Hormonal therapy | Aromatase inhibitors | aromatase enzyme | Anastrozole | Arimidex | Stops the activity of the aromatase enzyme, which produces estrogen, hence lowers the amount of estrogen in the body. | PO. | 1 mg daily. |
| | | | Exemestane | Aromasin | | PO, | 25 mg daily. |
| | | | Letrozole | Femara | | PO. | 2.5 mg daily. |
| | SERMs (elective estrogen-receptor modulators) | Estrogen receptor | Tamoxifen | Nolvadex | Blocks the receptor, hence blocking action of estrogen in the breast | PO. | 20-40 mg daily |
| | | | Raloxifene | Evista | | PO | 60 mg daily |
| | | | Toremifene | Fareston | | PO | 60 mg daily |
| | ERDs (Estrogenreceptor down-regulators) | | Fulvestrant | Faslodex | Block and break down estrogen receptors | IM | 250 mg once a month |
| | Progesterone inhibitor | Progesterone receptor | Megestrol acetate | Megace | Block progesterone receptor | PO | 160 mg daily |
| Chemotherapy | Alkylators | | Cyclophospham ide | Cytoxan | have a chemical structure that contain 2 alkyl groups that produce cross linking of the DNA which results in DNA breakage and tumor cell death | IV, IM, PO. | Varies. Common one: 100 mg/m2. |
| | Anti-metabolites | | Fluorouracil (5-FU) | Adrucil | act as false building blocks in a cancer cell's genes | IV | Varies. Common one: 600 mg/m2. |
| | | | Gemcitabine | Gemzar | | IV. | 1 g/m2 once a week |
| | | | Methotrexate | Trexall | | PO. Methotrexate -sodium: IV, IM, Intrathecal. | Varies. Common IV dosage: 40 mg/m2. |
| | Antibiotics | | Doxourbicin hydrochloride | Adriamycin | Thought to be related to the drug ability to bind DNA and inhibit nucleic acid synthesis | IV. | 60-75 mg/m2 once every 21 days as single chemo, 40-60 mg/m2 in combinations. Or 20 mg/m2 once a week. Or 30 mg/m2 daily for 3 days every 4 weeks. |
| | | | Epirubicin | Ellence | | | |
| | Antimiotic | | Vincristine | Oncovin | Vinca alkaloids act as antimicrotubule agents that block mitosis by arresting cells in the metaphase | | |
| | | | Vinorelbine | Navelbine | | IV | 20-30 mg/m2 once a week |
| | Anti-microtubule | | Paclitaxel | Taxol | Promotes the polymerization of tubulin, thereby causing cell death by disrupting the normal microtubule dynamics required for cell division | IV. | 100-250 mg/m2. |
| | | | Docetaxel | Taxotere | | | |
| | other | | Methotrexate | Trexall | | PO. Methotrexate -sodium: IV, IM, Intrathecal. | Varies. Common IV dosage: 40 mg/m2. |

## Claims

1. A cryosurgical probe (100, 200, 300) for providing an active substance in the vicinity of a tissue, comprising:
a. an injection unit, said injection unit comprising a chamber (106, 207, 308) for receiving the active substance, and a syringe needle (104, 208, 306) in communication with said chamber for administering the active substance to the tissue, further comprising a syringe barrel (112, 214, 313) connected to said chamber and a piston (113, 215, 314) for providing the active agent to said chamber; and
b. a cryoprobe unit for providing cryotherapy, said cryoprobe unit featuring a cryoneedle for insertion to the tissue, further comprising an inner lumen (101, 201, 303) for receiving cryogen and for cooling said cryoneedle, and an outer lumen for at least partially surrounding said injection unit; wherein said cryoprobe unit is combined with said injection unit in a single device and wherein said cryoprobe unit further comprises a longitudinal shaft (102, 301) for at least partially surrounding said inner lumen (101, 201, 303), said longitudinal shaft featuring a longitudinal groove (117, 219, 319) for receiving said syringe needle (104, 208, 306).

2. The probe of claim 1, wherein said cryoprobe unit further comprises a cryotip for sealing said outer lumen, said outer lumen featuring an opening adjacent to said cryotip for said needle to emerge, said cryotip causing said needle to be splayed away from said cryotip during forward displacement.

3. The probe of claim 2, further comprising a plurality of injection units and a plurality of needles, wherein said plurality of needles and said plurality of injection units are controlled with a single handle.

## Patentansprüche

1. Eine kryochirurgische Sonde (100, 200, 300) zur Abgabe eines Wirkstoffs in der Nähe eines Gewebes, bestehend aus:
a. einer Injektionseinheit, wobei besagte Injektionseinheit eine Kammer (106, 207, 308) zur Aufnahme des Wirkstoffs und eine Spritzennadel (104, 208, 306), die mit der besagten Kammer zur Verabreichung der aktiven Substanz in das Gewebe in Verbindung steht, umfasst, und überdies einen Spritzenzylinder (112, 214, 313), der mit der besagten Kammer verbunden ist, sowie einen Kolben (113, 215, 314) zur Verbringung des Wirkstoffs in die besagte Kammer, beinhaltet; und
b. einer Kryosondeneinheit zur Durchführung der Kryotherapie, wobei die besagte Kryosondeneinheit eine Kryonadel zum Einführen in das Gewebe aufweist, und überdies ein inneres Lumen (101, 201, 303) zur Aufnahme von Kryogen und zum Abkühlen der besagten Kryonadel sowie ein äußeres Lumen, das die besagte Injektionseinheit zumindest teilweise einschließt, umfasst; worin die besagte Kryosondeneinheit mit der besagten Injektionseinheit in einem einzelnen Gerät kombiniert wird und worin die besagte Kryosondeneinheit überdies einen Längsschaft (102, 301) beinhaltet, der das besagte innere Lumen (101, 201, 303) zumindest teilweise einschließt, wobei der besagte Längsschaft eine Längsnut (117, 219, 319) zur Aufnahme der besagten Spritzennadel (104, 208, 306) aufweist.

2. Die Sonde aus Anspruch 1, worin die besagte Kryosondeneinheit überdies eine Kryospitze zur Abdichtung des äußeren Lumens umfasst, wobei das besagte äußere Lumen eine an die besagte Kryospitze angrenzende Öffnung aufweist, damit die besagte Nadel zum Vorschein kommen kann, und die besagte Kryospitze bewirkt, dass die besagte Nadel von der besagten Kryospitze während der Vorwärtsbewegung weggespreizt wird.

3. Die Sonde aus Anspruch 2, die überdies eine Vielzahl von Injektionseinheiten und eine Vielzahl von Nadeln beinhaltet, worin die besagte Vielzahl von Nadeln und die besagte Vielzahl von Injektionseinheiten mit einem einzelnen Griff gesteuert werden.

## Revendications

1. Sonde cryogénique chirurgicale (100, 200, 300) destinée au passage d'une substance active dans le voisinage d'un tissu, comprenant :
a. une unité d'injection, ladite unité d'injection comprenant une chambre (106, 207, 308) pour la réception de la substance active, et une aiguille de seringue (104, 208, 306) en communication avec ladite chambre pour l'administration de la substance active au tissu, comprenant en outre un corps de seringue (112, 214, 313) connecté à ladite chambre et un piston (113, 215, 314) pour le passage de l'agent actif à ladite chambre ; et
b. une unité de sonde cryogénique pour l'administration d'une cryothérapie, ladite unité de sonde cryogénique étant dotée d'une aiguille cryogénique prévue pour une insertion dans le tissu, comportant en outre une lumière intérieure (101, 201, 303) pour la réception du cryogène et pour le refroidissement de la dite aiguille cryogénique, et une lumière extérieure pour entourer au moins partiellement ladite unité d'injection ; dans laquelle la dite unité de sonde cryogénique est combinée avec ladite unité d'injection dans un dispositif unique et dans laquelle ladite unité de sonde cryogénique comprend en outre un axe longitudinal (102, 301) pour entourer au moins partiellement ladite lumière intérieure (101, 201, 303), ledit axe longitudinal comportant une rainure longitudinale (117, 219, 319) pour la réception de ladite aiguille de seringue (104, 208, 306).

2. Sonde de la revendication 1, dans laquelle ladite unité de sonde cryogénique comprend en outre une pointe cryogénique destinée à sceller ladite lumière extérieure, cette dernière étant dotée d'une ouverture adjacente à ladite pointe cryogénique pour que ladite aiguille émerge, ladite pointe cryogénique causant ladite aiguille à s'évaser de la pointe cryogénique durant un déplacement vers l'avant.

3. Sonde de la revendication 2, comprenant en outre une pluralité d'unités d'injection et une pluralité d'aiguilles, dans laquelle ladite pluralité d'aiguilles et ladite pluralité d'unités d'injection sont contrôlées par un bras unique.
